# EUROPEAN PATENT APPLICATION

(11) **EP 3 115 007 A1**
(43) Date of publication of application: **11.01.2017**
(21) Application number: 15176333.1
(22) Date of filing: 10.07.2015
(51) Int. Cl.: A61B 17/68

(54) **CRANIAL FIXING CLIP**

(71) Applicant: HTSE S.A., 2346 Luxembourg (LU)
(72) Inventor: GUALANDRIS, Stefano, 6565 MESOCCO (CH); VITALE, Laura, 21100 VARESE (IT)
(74) Representative: Mittler, Andrea

(57) **Abstract**

A cranial fixing clip (10) for fastening a cranial bone flap (5) and a cranial bone mass (4) of a skull during craniotomy surgical operation, said cranial fixing clip (10) comprises a first end portion (12) and a second end portion (13) spaced apart and joined via an intermediate element (11), said first end portion (12) comprises a first tab portion (14) which is punched in the first end portion (12) and said second end portion (13) comprises a second tab portion (15) which is punched in the second end portion (13), wherein end portions (12, 14) and tab portions (14, 15) pass from an initial configuration obtained by subjecting said cranial fixing clip (10) to determined requirements to a final configuration by subjecting said cranial fixing clip (13) to different determined requirements, mantaining said intermediate element (11) on a vertical geometrical plane (K).

## Description

The present invention relates to a cranial fixing clip.

Devices for fixing cranial flaps exist in prior art.

For example patent EP-1765203-B1 describes a device for fastening cranial bone flaps, used to secure bone fragments or flaps separated from cranial bone mass during surgical operation. Said device comprises external support means and internal support means, which are joined via an intermediate element consisting of a shape-memory material, with an initial configuration obtained by subjecting said element to determined temperature requirements, and once deformed, in order to apply the device between the flap and the cranial bone mass, and upon certain new temperature requirements. External support means and internal support means are parallel with each other for engaging the flaps and the cranial bone mass. Disadvantageously the intermediate element of the device for fixing cranial flaps occupy too much space between the flap and the cranial bone mass and it is not possible to match exactly the flap and the cranial bone mass.

To resolve the space problem between the flap and the cranial bone mass, patents US-8206425-B2, US-8226694-B2 and US-2013/0282011-A1 describe devices for fastening cranial bone flaps, each of them comprising external support means and internal support means which embrace the flaps of cranial bone. Said external support means and internal support means are joined via an intermediate element which is vertical and directed along a direction between outside and inside of the cranial bone mass. Said intermediate element can be a resilient element as described in US-8206425-B2 or a screw as described in US-8226694-B2 or a ratchet surface with ratchet teeth as described in US-2013/0282011-A1, wherein said ratchet surface with ratchet teeth is able to permit that the external support means slide from external towards inside along the vertical direction. Disadvantageously it is not simple to fix or, once in position, to remove the external and internal support means from the flap and the cranial bone mass.

Trying to resolve this disadvantageous, patent EP-1437098-A1 describes a device for fastening cranial bone flaps comprising external support means and internal support means which embrace the flaps of cranial bone. Said external support means and internal support means are joined via an intermediate element which is vertical and directed along a direction between outside and inside of the cranial bone mass. Said intermediate element comprises a transversal resilient element which can yield an elastic pulsing force between the flaps and cranial bone mass. Disadvantageously the pushing force of the transversal resilient element of the intermediate element yields a stress to the cranial bone mass and the flaps and cranial bone mass do not match exactly each other. Disadvantageously the support means are not secure, because they do not embrace completely the extremitiy of the cranial bone mass.

Patent WO-2006/008174-A1 describes a cranial fixing clip for fastening cranial bone flaps, used to secure bone fragments or flaps separated from cranial bone mass during surgical operation. Said cranial fixing clip comprises external support means and internal support means, which are joined via an intermediate element consisting of a shape-memory material, with an initial configuration obtained by subjecting said element to determined temperature requirements, and once deformed, in order to apply the device between the flap and the cranial bone mass, and upon certain new temperature requirements. Said cranial fixing clip comprises a sheet element shaped so as to exhibit a first and a second opposite ends spaced apart and connected to one another by a central segment. In the proximity of each end there being punched a tab, respectively a first tab in the first end and a second tab in the second end each overturned in the direction of the end opposed to that wherein said tab is obtained. The central segment is angled relative to the two ends that lie on two different parallel planes. Each upturned tab, in cooperation with end and with the central segment of the sheet element realises the clipping engagement of the bone portion edge. During the transition from one configuration to the other, the cranial fixing clip tends to recover the deformation imposed but the bone constraint prevents a complete recovery, allowing the clip to work in constrained shape recovery while disadvantageously the central segment autonomously adjusts to the bone profile configuration. The fact that the central segment adjusts itself to the bone profile configuration is disadvantageous because said central segment is a transversal resilient element which yields an elastic pulsing force between the flaps and cranial bone mass during the surgical operations. Disadvantageously the pushing force of the transversal resilient element yields a stress to the cranial bone mass and the flaps.

The object of the present invention is to create a simpler cranial fixing clip for easier securely fastening cranial bone flaps, used to secure bone fragments or flaps separated from cranial bone mass during craniotomy surgical operation, the cranial fixing clip has to fit entirely the space between flap and cranial bone mass occupying only a smaller space also during surgical operation to reduce time of surgical operation, the flaps and the cranial bone mass extremities have to be better secured by the cranial fixing clip, the cranial fixing clip must not exert a transversal stress towards the extremity of the cranial bone mass, the cranial fixing clip must only exert a vertical force to embrace the flap and the cranial bone mass.

In accordance with the invention, this object is achieved with a cranial fixing clip according to claim 1.

These and other features of the present invention will become increasingly apparent from the following detailed description of one of its non-limiting practical embodiment examples disclosed in the accompanying drawings, in which:
figure 1 shows a perspective view of cranial fixing clip;
figure 2 shows an upside view of the cranial fixing clip;
figure 3 shows a sectional view along lines III-III of figure 2 in a first configuration;
figure 4 shows the sectional view along the lines III-III of figure 2 in a configuration wherein the cranial fixing clip embrace flap and cranial bone mass.

With reference to the above-listed figures, a cranial fixing clip 10 for fastening a cranial bone flap 5 and a cranial bone mass 4 of a skull.

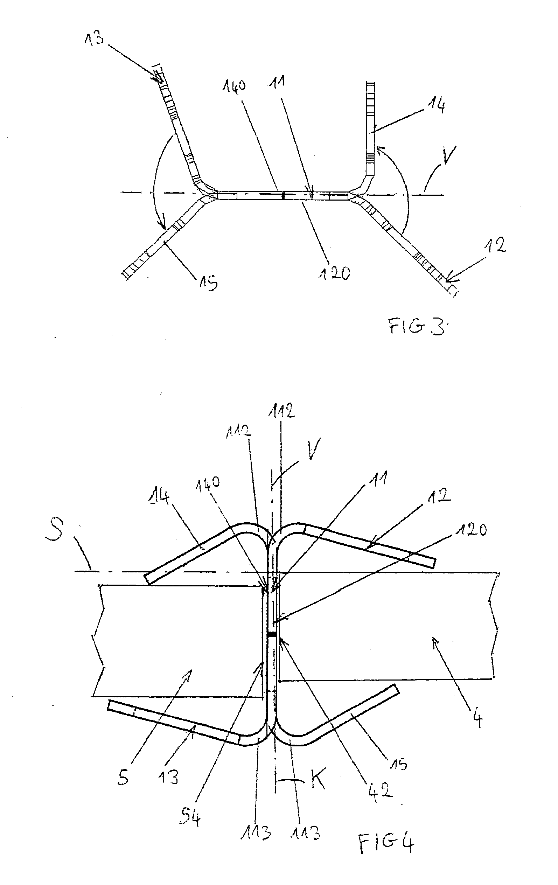

As shown in figure 4, said cranial bone flap 5 is separated from cranial bone mass 4 during craniotomy surgical operation. The separation between cranial bone flap 5 and cranial bone mass 5 creates an aperture of the skull. A thickness of the aperture of the cranial bone mass 4 identifies a vertical geometrical plane K which is perpendicular to a surface S of the skull.

Said cranial bone flap 5 comprises a vertical wall 54 along said vertical geometrical plane K and respectively, also said cranial bone mass 4 comprises a vertical wall 42 along said vertical geometrical plane K.

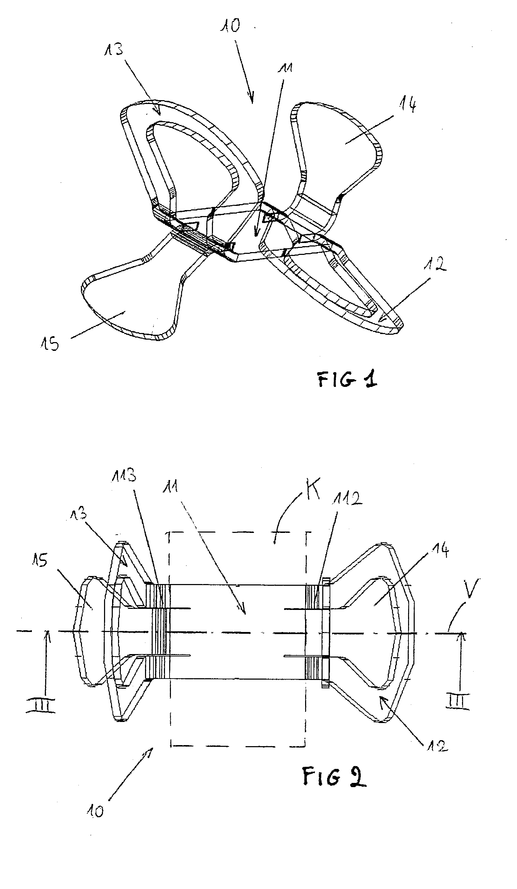

As shown in figure 1-4, said cranial fixing clip 10 consists of a sheet element 1.

Said sheet element 1 comprises a first end portion 12 and a second end portion 13 at the opposite side. The first end portion 12 and the second end portion 13 are spaced apart and joined via an intermediate element 11 of the sheet element 1.

Said first end portion 12 comprises a first tab portion 14 which is punched in the first end portion 12. The first tab portion 14 produces an opening in the first end portion 12.

Said second end portion 13 comprises a second tab portion 15 which is punched in the second end portion 13. The tab portion 15 produces an opening in the second end portion 13.

End portions 12, 14 and tab portions 14, 15 pass from an initial configuration obtained by subjecting said cranial fixing clip 10 to determined requirements to a final configuration by subjecting said cranial fixing clip 13 to different determined requirements.

The requirements depend from the nature of a shape-memory material.

Each one tab portion 14, 15 of said first tab portion 14 or second tab portion 15 passing from said initial configuration to said final configuration by bending overturned in the direction of the end portion 12, 13 opposed to that wherein said tab portion 14, 15 is obtained.

Each one end portion 12, 13 of said first end portion 12 or said second end portion 13 passing from said initial configuration to said final configuration by bending overturned in the opposite direction of the tab portion 14, 15 punched.

In the final configuration the first end portion 12 and the first tab portion 14 are external support means 12, 14 and the second end portion 13 and the second tab portion 15 are internal support means 13, 15, in the final configuration the first end portion 12 and the second tab portion 15 embrace said cranial bone mass 4, in the final configuration the first tab portion 14 and the second end portion 13 embrace said cranial bone flap 5 as it is shown in figure 4.

Said intermediate element 11 is flat and comprises a first surface 120 and a second surface 140.

Said intermediate element 11 is placed on said vertical geometrical plane K and remains on said vertical geometrical plane both in the initial configuration and in the final configuration.

In fact both in the initial configuration and in the final configuration each one surface 120, 140 of said first surface 120 and a second surface 140 of said intermediate element 11 is advantageously placed against and in contact with one vertical wall 42, 54 of the vertical wall 54 the cranial bone flap 5 or of the vertical wall 42 of the cranial bone mass 4. Thanks to the fact that both in initial and final configuration the surfaces 120 and 140 of said intermediate element 11 remains in place against and in contact with the respective vertical wall 42, 54 of the skull, the cranial fixing clip 10 advantageously fits entirely the space between flap 5 and cranial bone mass 4 occupying only a smaller space also during surgical operation. This advantageously reduces time of surgical operation and simplify the operation and the application of the cranial fixing clip 10 to the bones 4, 5 of the skull.

Said intermediate element 11 comprises a first bending portion 112 in direction of the first end portion 12 and a second bending portion 113 in direction of the second end portion 13. At least two bending portions 112, 113 of the cranial fixing clip 10 are made of the shape-memory material.

Said shape-memory material of the at least two bending portions 112, 133 yields passage of the two tab portions 14, 15 and the two end portions 12, 13 from the initial configuration to the final configuration. This simplify the cranial fixing clip 10 and permits to reduce the space between the cranial bones 4, 5 of the skull.

The requirements depends from the shape-memory material of the two bending portions 112, 113. The shape-memory material is selected from one of a list comprising NiTinol, Nickel-Titanium alloy, Copper Tantalum alloy, Iron Tantalum alloy, Vanadium Tantalum alloy, Niobium Tantalum alloy, Tungsten Tantalum alloy.

The present cranial fixing clip 10 is simpler than that of prior art for easier securely fastening cranial bone flaps, used to secure bone fragments or flaps separated from cranial bone mass during craniotomy surgical operation.

The cranial fixing clip 10 of the present invention is able to fix the flaps 5 and the cranial bone mass 4 extremities in a better and secured way, simplifying the surgical operation and being more handle for the user.

The cranial fixing clip 10 does not exert any transversal stress towards the extremity of the cranial bone mass 4. Thanks to the bending portions 112, 114 that deform themselves to make the tab portions 14, 15 and the end portions 12, 13 to pass from the initial to final configuration, maintaining the intermediate element 11 in vertical position, the cranial fixing clip 10 only exerts a vertical force to embrace the flap 5 and the cranial bone mass 4.

Alternatively all the cranial fixing clip 10 is made of shape-memory material, i.e. said sheet element 1 is all made of shape-memory material.

## Claims

1. Cranial fixing clip (10) for fastening a cranial bone flap (5) and a cranial bone mass (4) of a skull, wherein said cranial bone flap (5) is separated from cranial bone mass (4) during craniotomy surgical operation creating an aperture of the skull, wherein a thickness of the aperture of the cranial bone mass (4) identifies a vertical geometrical plane (K) which is perpendicular to a surface (S) of the skull, said cranial bone flap (5) comprises a vertical wall (54) along said vertical geometrical plane (K) and said cranial bone mass (4) comprises a vertical wall (42) along said vertical geometrical plane (K), said cranial fixing clip (10) consists of a sheet element (1), said sheet element (1) comprises a first end portion (12) and a second end portion (13) at the opposite side, the first end portion (12) and the second end portion (13) are spaced apart and joined via an intermediate element (11) of the sheet element (1), said first end portion (12) comprises a first tab portion (14) which is punched in the first end portion (12) and said second end portion (13) comprises a second tab portion (15) which is punched in the second end portion (13), wherein end portions (12, 14) and tab portions (14, 15) pass from an initial configuration obtained by subjecting said cranial fixing clip (10) to determined requirements to a final configuration by subjecting said cranial fixing clip (13) to different determined requirements, wherein each one tab portion (14, 15) of said first tab portion (14) or second tab portion (15) passing from said initial configuration to said final configuration by bending overturned in the direction of the end portion (12, 13) opposed to that wherein said tab portion (14, 15) is obtained, wherein each one end portion (12, 13) of said first end portion (12) or said second end portion (13) passing from said initial configuration to said final configuration by bending overturned in the opposite direction of the tab portion (14, 15) punched, wherein in the final configuration the first end portion (12) and the first tab portion (14) are external support means (12, 14) and the second end portion (13) and the second tab portion (15) are internal support means (13, 15), in the final configuration the first end portion (12) and the second tab portion (15) embrace said cranial bone mass (4), in the final configuration the first tab portion (14) and the second end portion (13) embrace said cranial bone flap (5), **characterized in that** said intermediate element (11) is flat and comprises a first surface (120) and a second surface (140), said intermediate element (11) is placed on said vertical geometrical plane (K) wherein both in the initial configuration and in the final configuration each one surface (120, 140) of said first surface (120) and a second surface (140) of said intermediate element (11) is placed against and in contact with one vertical wall (42, 54) of the vertical wall (54) the cranial bone flap (5) or of the vertical wall (42) of the cranial bone mass (4), said intermediate element (11) comprises a first bending portion (112) in direction of the first end portion (12) and a second bending portion (113) in direction of the second end portion (13), at least two bending portions (112, 113) of the cranial fixing clip (10) are made of shape-memory material, said shape-memory material of the at least two bending portions (112, 133) yields passage of the two tab portions (14, 15) and the two end portions (12, 13) from the initial configuration to the final configuration.

2. Cranial fixing clip (10) according to claim 1, **characterized in that** said sheet element (1) is all made of shape-memory material.

3. Cranial fixing clip (10) according to any one of the claims 1 or 2, **characterized in that** the shape-memory material is selected from one of a list comprising NiTinol, Nickel-Titanium alloy, Copper Tantalum alloy, Iron Tantalum alloy, Vanadium Tantalum alloy, Niobium Tantalum alloy, Tungsten Tantalum alloy.
